# EUROPEAN PATENT SPECIFICATION

(11) **EP 0 348 191 B1**
(45) Date of publication and mention of the grant of the patent: **23.02.1994**
(21) Application number: 89306290.1
(22) Date of filing: 22.06.1989
(51) Int. Cl.: G01N 33/537, G01N 33/551, G01N 33/554, G01N 33/555, G01N 33/80

(54) **Multiparameter particle analysis**
Teilchenanalyse auf mehrere Parameter
Analyse multiparamétrique de particules

(30) Priority: 23.06.1988 US 210688
(43) Date of publication of application: 27.12.1989
(73) Proprietor: SYNTEX (U.S.A.) INC., Palo Alto California 94303 (US)
(72) Inventor: Vorpahl, John, Livermore, CA 94550 (US); Ghazarossian, Vartan, Menlo Park, CA 94025 (US); Ullman, Edwin F., Atherton, CA 94025 (US)
(74) Representative: Armitage, Ian Michael

(56) References cited:
- EP-A- 0 121 442
- EP-A- 0 176 252
- DE-A- 3 617 763
- US-A- 4 267 235

## Description

The present invention relates to determination of specific binding members, for example antibodies.

The continued dependence upon whole blood obtained from individuals for replenishing blood in another person requires the monitoring of large numbers of blood samples for their blood group type. In determining the blood group, one is interested in a number of factors: The particular ABO group; the presence of antibodies to the antigens of the ABO group; and the Rh type. Where each of these factors must be determined independently, a large number of tests are involved. For the most part, hemagglutination tests have been involved in measuring the various factors, which are subjective labor intensive and cumbersome. Furthermore, instruments for automated testing are available but these instruments are expensive and designed for large numbers of tests. It is therefore desirable to find techniques which allow for minimal numbers of determination and automation of the method of determination, while accurately reporting the information necessary for blood typing.

Specific antibodies to cell surface antigens other than the A and B blood group antigens are commonly found in 1-2% of human blood samples. The presence of these antibodies in a blood transfusion recipient may cause an adverse reaction if the blood that is transfused contains cellular antigens complementary to the recipients antibodies. It is, therefore, necessary to test recipient blood for such antibodies. Normally, this is done by combining the serum or plasma of the recipient with "test cells" that are known to carry the relevant antigens on their surface. After incubation of this mixture, the cells are separated, washed free of the serum or plasma, and incubated with anti-immunoglobulin. This reagent, often called Coombs' reagent, binds to any recipient antibodies that are bound to the cells and causes the cells to agglutinate. Agglutination therefore indicates that the antibody screen is positive. Since the ability of the Coomb's reagent to cause agglutination can be blocked by patient immunoglobulin that is present if washing is incomplete, it is necessary to carry out a positive control when no agglutination of the test cells occurs. This may be done by separating the test cells from the solution containing the anti-immunoglobulin by centrifuging such solution and then adding to the solution "control cells" that are presensitized, that is, they have human antibodies bound to them. Usually, the control cells are prepared by incubating Rh positive cells with anti-Rh antibodies. If patient immunoglobulin has been removed and anti-immune globulin is present, the control cells will agglutinate and the test will be deemed valid. If they do not agglutinate, a negative antibody screen result indicated by the absence of agglutination the test cells is considered invalid.

The above described positive control for antibody screen requires that a solution containing the test cells be centrifuged or that the test cells be otherwise separated from the anti-immunoglobulin solution prior to addition of the control cells. This is considered necessary because of difficulty in observing test cell agglutination in the presence of control cells.

The present method avoids the difficulty of measuring agglutination of one cell type in the presence of another cell type and therefore permits the test cell positive control for antibody screening to be carried out without first removing the test cells from the suspension medium.

One aspect of the present invention is concerned with a method for determining the presence of a SBM bound to first particles (P₁) in a liquid medium. The method comprises providing in combination (i) a liquid medium suspected of containing SBM bound to P₁, (ii) means for agglutinating P₁ in relation to the presence of SBM on P₁ and (iii) second particles (P₂) having SBM or a different specific binding member for means for agglutinating bound thereto, thereby providing for means to agglutinate P₂. Agglutination of P₁ and P₂ are separately detectable and distinguishable by spectroscopic measurement. The medium is incubated and agglutination of each of the particles without separation is determined spectroscopically. Agglutination of P₁ is related to the presence of SBM on P₁ and agglutination of P₂ in the absence of agglutination of P₁ is related to the absence of SBM on P₁.

Another aspect of the present invention concerns a method for determining the presence of a specific binding member (SBM) in a liquid medium. The method comprises providing in combination in sequence a liquid medium suspected of containing SBM, (1) first particles having a specific binding member bound thereto (SBM₁-P₁), (2) means for agglutinating SBM₁-P₁, said means being reactive with SBM, and (3) second particles having a specific binding member for said means for agglutinating bound thereto (SBM₂-P₂), wherein at least one of SBM₁-P₁ or SBM₂-P₂ has a label, different labels and different particles being distinguishable by spectroscopic characteristics, which are emission, absorption and light scatter. The medium is incubated and at least a portion of said medium is irradiated with light, wherein said medium is continuous and said particles are suspended in said continuous medium. The populations of particles having electromagnetic signals differing from threshold values are determined and said populations are related to the presence of SBM.

Another aspect of the present invention concerns a method for determining the presence of an antibody in a sample. The sample suspected of containing the antibody is combined in an aqueous medium with first cells having a surface antigen reciprocal to the antibody. The cells are separated from the medium, combined in a second aqueous medium with an antibody for immunoglobulin, and incubated. Second cells having surface immunoglobulins recognizable by the antibody are subsequently added to the medium. At least one of the first or second cells has a fluorescent label. Different labels on different cells are distinguishable by spectroscopic characteristics which are emission, absorption, and light scattering. Agglutination of the first cells and the second cells is determined spectroscopically without separation of the cells from each other or from the second aqueous medium. The agglutination is then related to the presence of the antibody.

In accordance with the subject invention, methods are provided for determining agglutination of sets of particles in a medium with a minimum of determinations and separations of the particles from each other or from the medium. The methods have application for detecting agglutination of two sets of particles, for determining the presence of a specific binding member bound to particles, and particularly in the area of blood typing.

Before proceeding further with a description of the specific embodiments, the following terms will be defined.

Specific binding member ("SBM")--one of two different molecules, having an area on the surface or in a cavity which specifically binds to and is thereby defined as complementary with a particular spatial and polar organization of the other molecule. The specific binding member may be a ligand or receptor (antiligand). These will usually be members of an immunological pair such as antigen-antibody, although other specific binding pairs such as biotin-avidin, hormones-hormone receptors, nucleic acid duplexes, IgG-protein A, DNA-DNA, DNA-RNA, and the like are not immunological pairs but are included in the definition of SBM.

Ligand-any organic compound for which a receptor naturally exists or can be prepared.

Receptor ("antiligand")--any compound or composition capable of recognizing a particular spatial and polar organization of a molecule, e.g., epitopic or determinant site. Illustrative receptors include naturally occurring receptors, e.g., thyroxine binding globulin, antibodies, enzymes, Fab fragments, lectins, nucleic acids, protein A, complement component Clq, and the like.

Particles--the particles are generally at least about 0.02 µm and not more than about 100 µm, usually at least about 0.05 µm and less than about 20 µm, preferably from about 0.3 to 10 µm diameter. The particle may be organic or inorganic, swellable or non-swellable, porous or non-porous, preferably of a density approximating water, generally from about 0.7 to about 1.5 g/ml, and composed of material that can be transparent, partially transparent, or opaque. Normally, the particles will be biologic materials such as cells and microorganisms, e.g., erythrocytes, leukocytes, lymphocytes, hybridomas, streptococcus, Staphylococcus aureus, E. coli, viruses, and the like. The particles can also be particles comprised of organic and inorganic polymers, liposomes, latex particles, phospholipid vesicles, chylomicrons, lipoproteins, and the like.

Label--A member of the signal producing system. The label can be isotopic or non-isotopic, usually non-isotopic, including catalysts such as an enzyme, a chromogen such as a fluorescer, dye or chemiluminescer, a radioactive substance, and so forth, preferably a fluorescent dye.

Signal Producing System--The signal producing system may have one or more components, at least one component being a label. The signal producing system generates a signal that can be related to the presence or absence of an SBM in a sample. The signal producing system includes all of the reagents required to produce a measurable signal. Other components of the signal producing system can include substrates, enhancers, activators, chemiluminescent compounds, cofactors, inhibitors, scavengers, metal ions, specific binding substances required for binding of signal generating substances, and the like. Other components of the signal producing system may be coenzymes, substances that react with enzymic products, other enzymes and catalysts, and the like. The signal producing system provides a signal detectable by external means, preferably by measurement of electromagnetic radiation.

A large number of enzymes and coenzymes useful in a signal producing system are indicated in U.S. Patent No. 4,275,149, columns 19 to 23, and U.S. Patent No. 4,318,980, columns 10 to 14. A number of enzyme combinations are set forth in U.S. Patent no. 4,275,149, columns 23 to 28, which combinations can find use in the subject invention.

The fluorescers of interest will generally emit light at a wavelength above 350nm, usually above 400nm and preferably above 450nm. Desirably, the fluorescers have a high quantum efficiency, a large Stokes shift and are chemically stable under the conditions of their conjugation and use. The term fluorescer is intended to include substances that emit light upon activation by electromagnetic radiation or chemical activation and includes fluorescent and phosphorescent substances, scintillators, and chemiluminescent substances.

Fluorescers of interest fall into a variety of categories having certain primary functionalities. These primary functionalities include 1- and 2-aminonaphthalene, p,p-diaminostilbenes, pyrenes, quaternary phenanthridine salts, 9-aminoacridines, p,p′-diaminostilbenes, imines, anthracenes, oxacarbocyanine, merocyanine, 3-aminoequilenin, perylene, bis-benzoxazole, bis-p-oxazolyl benzene, 1,2-benzophenazine, retinal, bis-3-aminopyridinium salts, hellebrigenin, tetracycline, sterophenol, benzimidazolylphenylamine, 2-oxo-3-chromen, indole, xanthene, 7-hydroxycoumarin, 4,5-benzimidazoles, phenoxazine, salicylate, strophanthidin, porphyrins, triarylmethanes, flavin and rare earth chelates oxides and salts. Exemplary fluorescers are enumerated in U.S. Patent No. 4,318,707, columns 7 and 8. Squarine dyes described in U. S. Patent No. 4,806,488 are also useful as fluorescers.

A large number of illustrative fluorescers are indicated in U.S. Patent No. 4,275,149, columns 30 and 31.

Intervening removal--such removal refers to the separation of particles from a liquid medium such as by decantation, filtration, and the like and also includes centrifugation of the liquid medium to provide a centrifugate comprised of the particles and a supernatant liquid. In the latter instance the supernatant may or may not be physically separated from the centrifugate although the particles are removed from the liquid.

Means for agglutinating--means for causing particles to aggregate. Preferred means include specific binding members that are reciprocal to a specific binding member on a particle to be agglutinated. For example, where a particle contains a particular specific binding member (SBM) such as an antigen or antibody, the complementary specific binding member, preferably an antibody, can be employed to result in agglutination under appropriate conditions. When the SBM is an antibody, a preferred means for agglutinating is antibody for immunoglobulin. Antibodies can be produced by well-known techniques. Conditions for causing agglutination include incubation of the particles in an aqueous medium containing the means for aggutination, preferably with simultaneous gentle mixing to bring the particles into proximity with each other.

As mentioned above one aspect of the present invention involves a method for determining the presence of an SBM bound to first particles (P₁) in a liquid medium. A liquid medium suspected of containing SBM bound to P₁ is provided in combination with means for agglutinating P₁ in relation to the presence of the SBM on P₁ and second particles (P₂) having the same or a different specific binding member for said means for agglutinating bound thereto, thereby providing for means to agglutinate P₂. Agglutination of P₁ and P₂ are separately detectable and distinguishable by spectroscopic measurement.

The order of combining the above materials will usually provide for the liquid medium suspected of containing SBM bound to P₁ to first come into contact with the means for agglutinating P₁ and thereafter adding P₂ to that mixture. Preferably, the mixture will be incubated with mixing to provide for agglutination of P₁ prior to addition of P₂. The mixture will then again be incubated with mixing to provide for agglutination of P₂. Alternatively, all three materials can be combined simultaneously followed by incubation and mixing.

The medium will normally be aqueous, which may be up to about 40 weight percent of other polar solvents, particularly oxygenated solvents of from 1 to 6, more usually of from 1 to 4 carbon atoms, including alcohols, ethers and the like. Usually, the cosolvents will be present in less than about 20 weight percent. Under some circumstances depending on the nature of the sample, some or all of the aqueous medium could be provided by the sample itself.

After the materials are combined, the medium is incubated. The pH for the medium will usually be in the range of 4-11, more usually 5-10, and preferably in the range of about 6-9. The pH is chosen to maintain a significant level of binding affinity of the binding members to result in agglutination and also for optimal generation of signal by a signal producing system. Various buffers may be used to achieve the desired pH and maintain the pH during the assay. Illustrative buffers include borate, phosphate, carbonate, tris, barbital and the like. The particular buffer employed is not critical, but in individual assays, one buffer may be preferred over another. Moderate, and desirably substantially constant, temperatures are normally employed for the incubation and subsequent steps. The temperatures for the incubation and subsequent determination will generally be in the range of about 4°-50°C, more usually in the range of about 10°-40°C, and frequently will be ambient temperatures, that is, about 15°-25°C.

The concentration, in the liquid sample, of SBM or P₁ having SBM which may be determined will generally vary from about 10⁻⁴ to about 10⁻¹⁴M, more usually from about 10⁻⁶ to 10⁻¹⁴M. Considerations, such as the concentration of SBM and the protocol will normally determine the concentration of the other reagents.

While the concentrations of many of the various reagents and reagent solutions will generally be determined by the concentration range of interest of the SBM or P₁ having SBM, the final concentration of each of the reagents will normally be determined empirically to optimize the sensitivity of the determination over the range of interest. With certain protocols, individual reagents may be used in substantial excess without detrimentally affecting the sensitivity of the assay.

Following the incubation of the medium and particles, agglutination of each of the particles is determined spectroscopically. Generally, at least P₁ has a label bound thereto. By choice of label and agglutinating conditions at least two distinguishable signals are generated by the two agglutinated particles respectively. Usually, the signals from the two particles will be emission, absorption and/or light scattering.

The agglutination of P₁ is related to the presence of the SBM on P₁. The agglutination of P₂ in the absence of agglutination of P₁ is related to the absence of the SBM on P₁.

The method requires that the signal observed with agglutinated particles be different from the signal observed with non-agglutinated particles and that agglutination of P₁ gives a different signal than agglutination of P₂. Usually, when one particle is unlabeled and is observed by light scatter, it will be present in smaller quantity than the other particle, which will normally be labeled. Alternatively, the other particle can produce a different light scatter because it is a different size. When both particles are labeled with fluorescers, the labels will have different emission and/or absorption maxima or emission lifetimes. In certain cases, the label will naturally be present within the particle as, for example, chemiluminescence of lymphocytes and light absorption of erythrocytes. When the desired signal is light scatter, particles can be selected that have intrinsically high light scatter such as erythrocytes, colloidal metal, colloidal carbon, or colloidal dyes, etc. Labeling of a particle with a dye allows for detection of the labeled particle by absorption or divergence, usually divergence. In one method of detection a sufficiently small volume of the medium in which labeled particles are suspended is monitored such that on the average only one or a few particles will be inspected at a time. In order to determine if aggregation of the particles occurred, a large number of measurements of such measurement volumes can be made, which measurements differ temporally or spatially. When no aggregation has occurred, the number of particles within each volume, and hence the signal, will vary statistically and depend on the measurement volume and the concentration of the particles. When aggregation has occurred the presence of particle aggregates within some measurement volumes will increase the signal from those volumes and the frequency of measurements of signal exceeding a predetermined value will therefore be increased.

The sample will be continuous, being still or stirred or moving, usually moving relative to the measurement volume when the measurements differ temporally. Usually, the measurement volume will be contiguous with, and substantially surrounded by, the suspension medium and will be in a diffusive relationship with the entire sample being measured.

One means for irradiating measurement volumes is to employ a method and apparatus described in U.S. Patent No. 4,564,598. Basically, the measurement volume is irradiated employing an optical fiber where an irradiation volume that encompasses the measurement volume is determined by the construction of the optical fiber. The shape of the irradiation volume will normally be conical. The optical fibers are typically constructed of a core region and at least one cladding region, whose thickness (diameter) and relative refractive indices determine both the half angle of the cone and the cone's smallest diameter at the tip of the fiber. The effective axial length, that is, the length within which the measurement volume is located, is determined by the intensity of the excitation beam, the sensitivity of the detection methods and the rate of drop in intensity of the excitation light with increasing axial distance from the fiber tip. The rate depends upon the half angle of the cone, with larger half angles causing greater rates of intensity drop and, hence, shorter effective cone lengths. Also affecting the intensity drop will be light scattering and absorption properties of the medium.

The various parameters affecting the observed signal are chosen to insure that a measurable signal is available when a particle aggregate is present within the measurement volume, which signal will be significantly greater than the signal produced with no particles in the measurement volume.

Different effective measurement volumes can be provided by allowing for diffusion of particles in and out of the measurement volume. Alternatively, one can have a plurality of optical fibers, each one receiving signals from different irradiation volumes. Alternatively, a dynamic system may be used where the sample flows by one or more optical fibers or one or more optical fibers move through the sample.

The excitation light may be provided by irradiating the entire sample or a major portion of the sample with excitation light. Alternatively, and preferably, the excitation light may be provided by an optical fiber so that the measurement volume will be directly related to the volume irradiated.

The method of the present invention has particular application to the determination of the presence of an antibody in a sample. The method comprises combining in an aqueous medium the sample suspected of containing the antibody with first cells having a surface antigen reciprocal to the antibody. For example, in blood typing one determination involves the presence or absence of antibodies to the Lewis, Kell, and Duffy antigens and also to the Rh or D antigen. The sample may be serum, plasma or whole blood. The first cells having a surface antigen reciprocal to the antibody can be erythrocytes having the complementary antigen to the antibody being detected. The cells are then separated from the medium and combined in a second aqueous medium with antibody for an immunoglobulin. Usually, an incubation period follows. Second cells having surface immunoglobulins recognizable by the antibody for immunoglobulin are then added. At least one of the first or second cells has a fluorescent label and usually each has a different fluorescent label when the sample is whole blood. Different labels and different cells are distinguishable by spectroscopic characteristics, which include emission, absorption, and light scattering. The second aqueous medium is incubated. Agglutination of the first cells and the second cells is determined spectroscopically after each of the incubations or after the final incubation without intervening removal of the cells from the second aqueous medium. The agglutination is related to the presence of the antibody. Agglutination of the first cells indicates the presence of the antibody and agglutination of the second cells in the absence of agglutination of the first cells indicates the absence of the antibody.

As mentioned above, the present invention has particular application to automated blood typing procedures. It is useful in the Coombs antiglobulin test where immunoglobulin-containing plasma is first combined with test cells, which are then removed and carefully washed free of sample immunoglobulin prior to determinating whether antibody from the plasma has become bound to the cells by monitoring for agglutination upon addition of antiglobulin. The present invention allows the use of positive control cells to determine if washing is complete without intervening removal of the test cells from the antiglobulin containing medium or from the control cells.

In such a determination a sample comprising serum or plasma is combined and incubated with test cells that bear a fluorescent label and have surface-bound antigen reciprocal to the antibody to be determined. The test cells are then separated from the medium and washed so that the cells are free from the immunoglobulin from the sample. The test cells are then combined with antibody for immunoglobulin. The medium is incubated and agglutination is then determined. Control cells that have immunoglobulin on the surface are added prior to or following the determination. Preferably, the control cells will not be labeled or will be labeled with a different fluorescer. After another incubation measurement is made of agglutination of the control cells and, where not previously measured, of the test cells without intervening removal of the cells from the second aqueous medium. Agglutination of the test cells indicates the presence of antibody in the sample. Agglutination of the control cells in the absence of agglutination of the test cells indicates the absence of the antibody in the sample.

Failure of the test cells to agglutinate when the antibody to be determined is present can result when immunoglobulin from the sample is present in the medium because of incomplete washing after the initial separation. Control cells are used to determine the completeness of the wash. If the control cells agglutinate, then the initial wash has removed sufficient immunoglobulin to avoid absorption of the added antibody for immunoglobulin. The antibodies for immunoglobulin are therefore available to agglutinate the test cells when immunoglobulin has become bound to the test cells. Consequently, failure of the test cells to agglutinate when the control cells have agglutinated indicates that the antibody to be determined was not in the sample. The agglutination can be carried out in one liquid medium without intervening separation of the test cells from the medium or from the second cells.

Another aspect of the present invention concerns a method for determining the presence of a SBM in the liquid medium. A liquid medium suspected of containing the SBM is provided in combination with (1) first particles having a specific binding member complementary to SBM bound thereto (SBM₁-P₁), and (2) means for agglutinating SBM₁-P₁, wherein the means are reactive with SBM; incubating the mixture; and adding second particles having a specific binding member for the means for agglutination bound thereto (SBM₂-P₂). At least one of SBM₁-P₁ or SBM₂-P₂ has a label. The different labels and different particles are distinguishable by spectroscopic characteristics, which are emission, absorption and light scatter.

Referring to the above particular example of the use of test cells in blood typing, the SBM is exemplified by a patient's immunoglobulin. As mentioned above, it is desirable to determine whether all of the patient's immunoglobulin has been removed in the first wash. SBM₁-P₁ is exemplified by cells having an antigen to which the antibody being determined in the blood typing is attached. SBM₂-P₂ is exemplified by the control cells and the means for agglutinating is exemplified by antibody to immunoglobulin.

The medium is then incubated and at least a portion of the medium is irradiated with light. The medium is continuous and the particles are suspended in the continuous medium. Populations of particles having electromagnetic signals differing from threshold values are determined and related to the presence of SBM. In one approach the SBM₁-P₁ is labelled with a fluorophor and the method involves determining emission and light scatter. Agglutination of SBM₁-P₁ indicates the presence of SBM bound to this particle. Agglutination of SBM₂-P₂ in the absence of agglutination of SBM₁-P₁ indicates the absence of SBM bound to SBM₁-P₁.

For convenience, the reagents for conducting a method in accordance with the present invention can be provided in a kit. The kit comprises the reagents in packaged combination. The reagents are packaged according to their respective reactivities to one another and to insure shelf life. For example, each reagent can be packaged in separate containers or reagents can be combined where co-reactivity will permit. In the area of blood typing the reagents will include test cells carrying a label, control cells, and antibody for immunoglobulin. The kit can also include ancillary materials such as buffers and stabilizers. In addition, other ancillary materials include proteins such as albumins, surfactants, particularly non-ionic surfactants, binding enhancers, e.g., polyalkylene glycols, and the like.

The invention is demonstrated further by the following illustrative examples. Parts and percentages are by volume unless indicated otherwise.

### EXAMPLE 1

### Cell Detection System

The light beam for a He/Ne laser (2 mW, Melles Griot, San Marcos, CA) emitting at 633 nm was focused onto one end of a pure silica optical fiber having a 50 µm core and a numerical aperture of 0.22. The other end of the fiber was incorporated into a dual fiber probe designed such that the axis of the cone of light emanating from the end of the fiber intersected at a 60° angle with the axis of a second like fiber at a distance of about 225 µm from the end of each fiber. The collection volume, defined as the overlap of the light cone from the first fiber and the light acceptance cone of the second fiber, was about 10⁻⁶ cm³. The probe was designed to be immersed directly into the sample to be analyzed. A portion of the light that either was emitted by fluorescent particles or solutes or was scattered from particles within the collection volume could be transmitted by the second fiber and focused through a filter wheel onto a photomultiplier (Hamamatsu, Bridgewater, NJ). The filter wheel contained a 633 nm bandpass filter for detection of scattered light and a tandem pair of 645 nm long-pass filters for detection of fluorescent light. The photomultiplier output was processed through a preamplifier and discriminator (Pacific Instruments, Concord, CA) resulting in a photopulse output suitable for photon counting. Photopulses were counted over 500 µs gate times and the count passed to an on-line microprocessor. Fluctuations of the counts in sequential gate times were used to estimate the degree of aggregation of particles.

### Sample transport and mixing

Samples and reagents were combined in a single drop on a flat sheet of latex rubber held in contact with the upper surface of a temperature-controlled reaction block. Within the block underlying the latex sheet were cup-shaped cavities that were about 1 cm in diameter that were each connected to a vacuum line through a 1 mm opening at the bottom. Efficient mixing of the drops was achieved by alternately evacuating the cups to cause a section of the sheet to be stretched and drawn downward, and releasing the vacuum to permit the sheet to return to its original position. With sufficiently small liquid volumes, the drops of reaction mixtures remained in place by surface tension and could be transported across a series of cavities by moving the sheet during periods when no vacuum was applied. This permitted the addition and removal of reagents at different locations and made it possible to simultaneously process a series of samples. The final cavity in the series was positioned under the dual fiber probe which was equipped with a dispenser for dilution of the cells and could be moved vertically to permit immersion into the suspension.

### Dyes

1-3-bis [4-dibutylamino-2-hydroxyphenyl]-2,4-dihydroxycyclobutenediylium dihydroxide, bis (inner salt) (TBS) was used to stain red blood cells. The dye was synthesized by the method of Sprenger and Ziegebein, Angew. Chem., Internat. Edit. (1966) 5: 894, by refluxing one equivalent of squaric acid with two equivalents of N,N-dibutylaniline in a 2:1 mixture of n-butanol and benzene with continuous azeotropic removal of water. On cooling, the products separated as green crystals which were collected by filtration and washed with methanol. The dyes were recrystallized from a 1:1 mixture of methanol and methylene chloride. The absorption maximum of the dye in dimethylformamide was 650 nm, ε = 330,000 M⁻¹ cm⁻¹.

### Antibody Reagents

Anti-human IgG was prepared from the serum of rabbits immunized with human IgG (Mollison, "Blood Transfusion in Clinical Medicine" (7th edition), Blackwell Scientific Publications (1983), 505-508. The IgG fraction was isolated by ammonium sulfate precipitation and ion exchange chromatography (DEAE-Sephacell) and diluted in 20 mM Tris-saline buffer to 11-18 mg/mL with 1.25% PVP.

### Reagent Red Blood Cells

For antibody screening, cells were obtained from three different donors which were selected such that each of the approximately 20 clinically significant antigens were represented on one or more of the cells. The cells were washed with saline and suspended to approximately 2 x 10⁹ cells/mL in saline containing 2% BSA. Fifty µL of 10⁻⁴ M TBS in dimethylacetamide were added to one mL of this suspension and the mixture was gently mixed for 15-30 minutes at room temperature. The cells were then washed with saline and stored at 4°C in an isotonic cell medium (pH 8.3) (Uda, et al., Transfusion (A85) 25:325-329) at a final concentration of 5% (v/v) for reverse grouping cells and 10% for antibody screening cells.

Control cells used for the antibody screen (Uda, et al., supra) were prepared by incubating Rh(D) positive cells with a human monoclonal anti-Rh(d) antibody (Bioresponse, 0.24 µg/mL) in saline with 2% BSA for 45 minutes at 37°C. The antibody coated cells were washed in saline and stored in cell medium (Widmann, et. "Technical Manual," 9th ed., Arlington, VA, American Association of Blood Banks (1985) 376) at a concentration of 10% (v/v).

### Ferrofluid

Colloidal magnetizable magnetite was prepared by a modification of the procedure described by Massart (IEEE Transactions on Magnetics, MAG 17 (1981) 1247). Equal volumes of 1.5 M ammonium hydroxide and an aqueous solution containing 0.16 M FeCl₂, 0.08 M FeCl₂, and 0.08 M HCl at room temperature were pumped into a flask over a 5 minute period. After the addition was complete, the magnetite was allowed to settle for one hour and the supernatant, which represented about 80% of the total volume, was decanted. The concentrated slurry of magnetite and an equal volume of 2.0 M perchloric acid were simultaneously pumped into a second flask. The suspended material was separated magnetically. Upon addition of one-fifth the original volume of water, a colloidal dispersion was obtained which was dialyzed against 10 mM perchloric acid. The resulting suspension contained 44 mg/mL of iron as determined by addition of an equal volume of concentrated hydrochloric acid and assaying according to the method of Persijn, et al. (Clinica Chimica Acta (1971) 35: 91-98).

Succinylated BSA was prepared by adding 2.5 mL of 0.5 M succinic anhydride (Aldrich) in dimethylformamide (Mallinckrodt) to 5.0 g of BSA (crystalline grade, Miles Laboratories) in 250 mL of 0.1 M sodium phosphate, pH 8.0, at room temperature. After 1.5 hours, the solution was dialyzed exhaustively against deionized water. The electrophoretic mobility of the modified protein was 1.75 (native BSA = 1.00), determined by agarose gel electrophoresis (Paragon, SPE, Beckman).

The colloidal iron oxide was diluted with water to 10 mg/mL (pH 2.9) and 100 mL of this suspension was combined with an equal volume of succinylated BSA (9.5 mg/mL, adjusted to pH 3.3 with 0.1 M HCl0₄). The pH of the solution was then raised to 9.0 by the rapid addition of 15-20 mL of 0.1 M tetramethylammonium hydroxide. During this process, the clear, dark-brown liquid became cloudy and then clarified again. The average particle size was determined by dynamic scattering spectrometry (Nicomp Instruments, Model HM5-90) to be 55-65 nm. A portion of the ferrofluid preparation was concentrated 5-fold by membrane ultrafiltration (Amicon) to prepare a 25 mg/mL reagent.

### Other Reagents

Saline was an aqueous solution of 0.15 NaCl. Low-ionic-strength-solution (LISS) was an isotonic phosphate-buffered glycine solution containing 0.03 M NaCl, pH 6.7. Polyacrylic acid (Aldrich, ave. MW = 5000) was diluted to 5 mg/mL in 0.1M glycine buffer, pH 8.0. Polyvinylpyrrolidone (PVP) (GAF) was K-60, nonpharmaceutical grade. Polybrene® (hexadimethrine bromide, Aldrich, ave MW = 4300) was a 16 mg/mL solution in LISS, pH 6.7.

### Automated Antibody Screening

Plasma was first removed from the blood to be tested by mixing 0.5 mL of blood, 40 µL of 25 mg/mL ferrofluid, 200 µL of LISS, and 40 µL of polybrene in a 1 cm² cup. The cell-ferrofluid coaggregates were separated by positioning like poles of two 0.21 tesla (2.1 kgauss) magnets as close as possible to adjacent sides of the cup.

The cell-free plasma was removed and tested against three different sets of stained reagent red blood cells. For each test, 10 µL of cells and 100 µL of plasma were mixed for 9 minutes on the latex sheet at a reaction block temperature of 37°C. Twenty microliters of ferrofluid (5 mg/mL), 20 µL of polybrene, and 50 µL LISS were then added and the cells magnetically separated. The liquid was removed and the cells washed with LISS and resuspended by addition of 15 µL of polyacrylic acid. Anti-human IgG reagent (20 µL) was then added and the cell suspension was mixed for 1 minute. Next, control cells (10 µL) were added, and after 1 minute of incubation, the sample was diluted with 0.5 mL of saline. The resulting suspension was analyzed sequentially for agglutination of the reagent cells (fluorescence) and the control cells (light scatter).

### Interpretation of Results

Agglutination of erythrocytes was determined by drawing the cell suspension past the end of the dual fiber probe and monitoring the fluorescence or light scatter in multiple sample volumes defined by 500 µs gate times. Continuous measurements were recorded for 10 seconds. The data were analyzed for first identifying series of sequential gate times that had light pulse counts in excess of a threshold value and were therefore attributed to the passage of one or more cells past the probe. Signal pulses were usually about 3-4 gate times (1.5-2 ms) depending on the rate of motion of the liquid. These events were counted to provide an "agglutination index." For the purpose of distinguishing an agglutinated from a nonagglutinated sample, "cut-off" agglutination index values were obtained by testing known samples.

In the antibody screen test, increased fluctuations in the fluorescent signal above a "cut-off" agglutination index were attributed to agglutination of the stained cells. The fluorescence signal was monitored by using the 645 nm long-pass filter. The antibody screen control cells were not stained, and agglutination was therefore monitored by measurement of fluctuations in the light scatter signal using the 633 nm band-pass filter. Since these cells were coated with human IgG, their agglutination served as an indicator that enough anti-human IgG had been added. Thus, a sample that tested negative in the antibody screen was considered to be indeterminant unless agglutination of the control cells was detected by light scatter fluctuations.

### RESULTS

### Automated Testing

The automated antibody screen was performed on 103 specimens that were either fresh blood or were "reconstituted" samples prepared by mixing antibody-positive plasma (stored frozen) with compatible red blood cells to stimulate a positive sample. The samples had a broad range of titers of antibodies to a variety of clinically significant antigens. In the automated method, plasma obtained by magnetic separation was tested against a panel of three sets of antibody screen reagent cells. Each sample was also tested with the same reagent cells using standard manual techniques and reagents. The results are summarized in Table 1. Two of 26 samples that tested negative by the manual method were positive by automated testing, and 7 of 77 samples that were positive by the manual method were negative by the automated method. The seven samples that were missed by the automated method produced very weak but reproducible agglutination by manual testing. The one indeterminate sample failed to show a light scatter signal from the control cells, due presumably to incomplete removal of IgG during washing of the cells.

**Table 1**

| Correlation between manual and automated antibody screening of 103 patient samples. | | | |
|---|---|---|---|
| Manual Determination | Automated Screen | | |
| | Positive | Negative | Indeterminate^{a} |
| Positive | 69 | 7 | 1 |
| Negative | 2 | 24 | 0 |

| | | | |
|---|---|---|---|
| ^{a} Lack of agglutination of the control cells. | | | |

The present method takes advantage of the clinically proven agglutination chemistry while providing novel separation, sample handling, and detection methods to permit automation and objective interpretation. The method permits antibody screening involving the use of test cells and control cells to be carried out without the need for a separation step such as centrifugation, decantation, and the like.

Although the foregoing invention has been described in some detail by way of illustration and example for purposes of clarity of understanding, it will be obvious that certain changes and modifications may be practiced within the scope of the appended claims.

## Claims

1. A method for determining the presence of a specific binding member (SBM) bound to first particles (P₁) in a liquid medium, said method comprising:
providing sequentially in combination (1) a liquid medium suspected of containing SBM bound to P₁, (2) means for agglutinating P₁ in relation to the presence of said SBM on P₁, and (3) second particles (P₂) having the same or a different specific binding member for said means for agglutinating bound thereto, thereby providing for said means to agglutinate P₂, wherein agglutination of P₁ and P₂ are separately detectible and distinguishable by spectroscopic measurement;
incubating said medium;
determining spectroscopically agglutination of each of said particles without separation of P₁ from P₂; and
relating said agglutination of P₁ to the presence of said SBM on p₁ and agglutination of P₂ in the absence of agglutination of P₁ to the absence of said SBM on P₁.

2. The method of Claim 1 wherein said SBM is an immunoglobulin.

3. The method of Claim 1 wherein said first particles are cells having a surface antigen with an antibody bound thereto.

4. The method of Claim 1 wherein said means for agglutinating is antibody for immunoglobulin.

5. The method of Claim 1 wherein said second particles are cells having surface immunoglobulins.

6. The method of Claim 1 wherein said first particle has a label bound thereto.

7. The method of Claim 6 wherein said label is a fluorophor.

8. The method of Claim 1 wherein said determining involves emission and light scatter.

9. A method for determining the presence of an antibody in a sample, said method comprising:
combining in an aqueous medium a sample suspected of containing an antibody of interest with first cells having a surface antigen reciprocal to said antibody;
separating said cells from said medium;
combining in a second aqueous medium said cells and an antibody for an immunoglobulin,
incubating said second aqueous medium and adding to it second cells having surface immunoglobulins recognizable by said antibody for an immunoglobulin wherein at least one of said first or second cells has a fluorescent label, different labels and different cells being distinguishable by spectroscopic characteristics, which are emission, absorption, and light scattering,
determining spectroscopically agglutination of said first cells and said second cells without separation of said cells from each other or from said second aqueous medium; and
relating said agglutination to the presence of said antibody of interest.

10. The method of Claim 9 wherein said antibody is selected from the group consisting of immunoglobulins specific to human erythrocyte surface antigens.

11. The method of Claim 9 wherein said immunoglobulins on said second cells have the same binding properties as said antibody in said blood sample.

12. The method of claim 9 wherein said fluorescent label is selected from the group consisting of fluorescein and rhodamine derivatives, phycobiliproteins, squaraines, umbelliferones, cyanines and merocyanines.

13. The method of Claim 9 wherein said first and second cells have different fluorescent labels bound thereto.

14. The method of Claim 9 wherein said first cells are labeled and said determining involves emission and light scatter.

15. A method according to claim 9 wherein said sample is a blood sample,
and the spectroscopic determination comprises
irradiating at least a portion of said medium with light, wherein said medium is continuous and said cells are suspended in said continuous medium, and determining populations of cells having electromagnetic signals differing from threshold values; and
relating said populations to the presence of said antibody.

## Patentansprüche

1. Verfahren zur Bestimmung der Anwesenheit eines spezifischen bindenden Mitglieds (SBM), das in einem flüssigen Medium an erste Teilchen (P₁) gebunden ist, wobei das Verfahren umfaßt:
das in Kombination nacheinander Bereitstellen (1) eines flüssigen Mediums, von dem man vermutet, daß es SBM an P₁ gebunden enthält, (2) Mittel zum Agglutinieren von P₁ in Beziehung zur Anwesenheit des SBM auf P₁ und (3) zweite Teilchen (P₂), die das gleiche oder ein verschiedenes spezifisches bindendes Mitglied für das Mittel zum Agglutinieren an sie gebunden aufweisen, wodurch sie dafür sorgen, daß das Mittel P₂ agglutiniert, worin Agglutination von P₁ und P₂ durch spektroskopische Messung getrennt nachweisbar und unterscheidbar sind;
das Inkubieren des Mediums;
das spektroskopische Bestimmen von Agglutination jedes der Teilchen ohne Trennung von P₁ von P₂; und
das Inbeziehungsetzen der Agglutination von P₁ zu der Anwesenheit des SBM auf P₁ und der Agglutination von P₂ in Abwesenheit von Agglutination von P₁ zu der Abwesenheit des SBM auf P₁.

2. Verfahren nach Anspruch 1, worin das SBM ein Immunglobulin ist.

3. Verfahren nach Anspruch 1, worin die ersten Teilchen Zellen sind, die ein Oberflächenantigen mit einem daran gebundenen Antikörper aufweisen.

4. Verfahren nach Anspruch 1, worin das Mittel zur Agglutination Antikörper gegen Immunglobulin ist.

5. Verfahren nach Anspruch 1, worin die zweiten Teilchen Zellen mit Oberflächen-Immunglobulinen sind.

6. Verfahren nach Anspruch 1, worin das erste Teilchen einen an es gebundenen Marker aufweist,

7. Verfahren nach Anspruch 6, worin der Marker ein Fluorophor ist.

8. Verfahren nach Anspruch 1, worin die Bestimmung Emission und Lichtstreuung beinhaltet.

9. Verfahren zur Bestimmung der Anwesenheit eines Antikörpers in einer Probe, wobei das Verfahren umfaßt:
das Vereinigen einer Probe, von der man annimmt, daß sie einen interessierenden Antikörper enthält, mit ersten Zellen, die ein zu dem Antikörper reziprokes Oberflächenantigen aufweisen, in einem wäßrigen Medium;
das Abtrennen der Zellen aus dem Medium;
das Vereinigen der Zellen und eines Antikörpers gegen ein Immunglobulin in einem zweiten wäßrigen Medium,
das Inkubieren des zweiten wäßrigen Mediums und die Zugabe zu demselben von zweiten Zellen mit für den Antikörper gegen ein Immunglobulin erkennbaren Oberflächen-Immunglobulinen, worin mindestens eine der ersten oder zweiten Zellen einen fluoreszierenden Marker aufweist, wobei verschiedene Marker und verschiedene Zellen durch spektroskopische Eigenschaften unterscheidbar sind, welche Emission, Absorption und Lichtstreuung sind,
das spektroskopische Bestimmen von Agglutination der ersten Zellen und der zweiten Zellen ohne Abtrennung der Zellen voneinander oder aus dem zweiten wäßrigen Medium; und
das Inbeziehungsetzen der Agglutination zur Anwesenheit des interessierenden Antikörpers.

10. Verfahren nach Anspruch 9, worin der Antikörper aus der Gruppe ausgewählt ist, die aus für Humanerythrozyten-Oberflächenantigene spezifischen Immunglobulinen besteht.

11. Verfahren nach Anspruch 9, worin die Immunglobuline auf den zweiten Zellen die gleichen Bindungseigenschaften wie der Antikörper in der Blutprobe aufweisen.

12. Verfahren nach Anspruch 9, worin der fluoreszierende Marker aus der Gruppe ausgewählt ist, die aus Fluorescein- und Rhodamin-Derivaten, Phycobiliproteinen, Quadratainen, Umbelliferonen, Cyaninen und Merocyaninen besteht.

13. Verfahren nach Anspruch 9, worin die ersten und zweiten Zellen unterschiedliche an sie gebundene fluoreszierende Marker aufweisen.

14. Verfahren nach Anspruch 9, worin die ersten Zellen markiert sind und die Bestimmung Emission und Lichtstreuung beinhaltet.

15. Verfahren nach Anspruch 9, worin die Probe eine Blutprobe ist und die spektroskopische Bestimmung das Bestrahlen mindestens eines Teils des Mediums mit Licht, worin das Medium kontinuierlich ist und die Zellen in dem kontinuierlichen Medium suspendiert sind, und das Bestimmen von Populationen von Zellen mit elektromagnetischen Signalen, die von Schwellenwerten verschieden sind, umfaßt; und
das Inbeziehungsetzen der Populationen zur Anwesenheit des Antikörpers.

## Revendications

1. Méthode pour déterminer la présence d'un membre à liaison spécifique (MLS) lié à des premières particules (P₁) dans un milieu liquide, ladite méthode comprenant:
la mise en association, successivement, (1) d'un milieu liquide suspecté de contenir un MLS lié à P₁, (2) d'un moyen d'agglutination de P₁ en rapport avec la présence dudit MLS sur P₁, et (3) de secondes particules (P₂) portant un membre à liaison spécifique, identique ou différent pour ledit moyen d'agglutination, lié à ces particules, ce qui permet audit moyen de provoquer l'agglutination de P₂, les agglutinations de P₁ et P₂ étant détectables et différenciables séparément par une mesure spectroscopique ;
la mise en incubation dudit milieu ;
la détermination spectroscopique de l'agglutination de chacune desdites particules sans séparer P₁ de P₂ ; et
l'établissement de la relation entre ladite agglutination de P₁ et la présence dudit MLS sur P₁, et entre l'agglutination de P₂ en l'absence d'agglutination de P₁ et l'absence dudit MLS sur P₁.

2. Méthode suivant la revendication 1, dans laquelle le MLS est une immunoglobuline.

3. Méthode suivant la revendication 1, dans laquelle les premières particules sont des cellules portant un antigène de surface auquel est lié un anticorps.

4. Méthode suivant la revendication 1, dans laquelle le moyen d'agglutination est un anticorps pour une immunoglobuline.

5. Méthode suivant la revendication 1, dans laquelle les secondes particules sont des cellules portant des immunoglobulines de surface.

6. Méthode suivant la revendication 1, dans laquelle la première particule porte un marqueur lié à cette particule.

7. Méthode suivant la revendication 6, dans laquelle le marqueur est un fluorophore.

8. Méthode suivant la revendication 1, dans laquelle la détermination fait intervenir une émission et une diffusion de lumière.

9. Méthode de détermination de la présence d'un anticorps dans un échantillon, ladite méthode comprenant :
l'association dans un milieu aqueux d'un échantillon suspecté de contenir un anticorps intéressant avec des premières cellules portant un antigène de surface correspondant audit anticorps ;
la séparation desdites cellules dudit milieu ;
l'association dans un second milieu aqueux desdites cellules et d'un anticorps pour une immunoglobuline,
la mise en incubation dudit second milieu aqueux et l'addition à ce second milieu aqueux le secondes cellules portant des immunoglobulines de surface reconnaissables par ledit anticorps pour une immunoglobuline, au moins un type desdites premières et secondes cellules portant un marqueur fluorescent, des marqueurs différents et des cellules différentes étant différenciables par les caractéristiques spectroscopiques, qui consistent en une émission, une absorption et une diffusion de lumière,
la détermination par spectroscopie de l'agglutination desdites premières cellules et desdites secondes cellules sans séparation desdites cellules les unes des autres ou dudit second milieu aqueux ; et
l'établissement de la relation entre ladite agglutination et la présence dudit anticorps intéressant.

10. Méthode suivant la revendication 9, dans laquelle l'anticorps est choisi dans le groupe consistant en immunoglobulines spécifiques des antigènes de surface d'érytrocytes humains.

11. Méthode suivant la revendication 9, dans laquelle les immunoglobulines sur les secondes cellules possèdent les mêmes propriétés de liaison que l'anticorps dans l'échantillon de sang.

12. Méthode suivant la revendication 9, dans laquelle le marqueur fluorescent est choisi dans le groupe consistant en dérivés de fluorescéine et de rhodamine, phycobiliprotéines, squaraines, umbelliférones, cyanines et mérocyanines.

13. Méthode suivant la revendication 9, dans laquelle les premières et secondes cellules portent des marqueurs fluorescents différents liés à ces cellules.

14. Méthode suivant la revendication 9, dans laquelle les premières cellules sont marquées et la détermination fait intervenir une émission et une diffusion de lumière.

15. Méthode suivant la revendication 9, dans laquelle l'échantillon est un échantillon de sang et la détermination spectroscopique comprend l'irradiation d'au moins une partie dudit milieu avec de la lumière, ledit milieu étant continu et les cellules étant mises en suspension dans ledit milieu continu, et la détermination des populations de cellules présentant des signaux électromagnétiques différents de valeurs seuils; et
l'établissement de la relation entre lesdites populations et la présence dudit anticorps.
